# EUROPÄISCHE PATENTSCHRIFT

(11) **EP 2 774 600 B1**
(45) Veröffentlichungstag und Bekanntmachung des Hinweises auf die Patenterteilung: **05.04.2017**
(21) Anmeldenummer: 14150905.9
(22) Anmeldetag: 13.01.2014
(51) Int. Cl.: A61K 8/29, A61Q 1/02, A61K 8/06, A61K 8/34, A61K 8/02

(54) **lamellare Öl-in-Wasser-Emulsionen mit mehrschichtigen Pigmentkapseln**
Lamellar oil-water emulsions with multilayer pigment capsules
Émulsions huile dans eau lamellaires avec capsules de pigments multicouches

(30) Priorität: 13.02.2013 DE 102013202330
(43) Veröffentlichungstag der Anmeldung: 10.09.2014
(73) Patentinhaber: Henkel AG & Co. KGaA, 40589 Düsseldorf (DE)
(72) Erfinder: Dickhof, Susanne, 41748 Viersen (DE); Keßler-Becker, Daniela, 51371 Leverkusen (DE)

(56) Entgegenhaltungen:
- WO-A1-2004/055119
- WO-A1-2013/106998
- WO-A1-2013/106999
- WO-A1-2013/107000
- DE-A1-102009 060 148
- US-A1- 2009 155 321
- US-A1- 2009 155 322

## Beschreibung

Die Erfindung betrifft Emulsionen, die eine "überraschende" Farbveränderung auf der Haut bewirken können.

Neben der Pflege und Glättung der Haut sowie dem Vorbeugen vor oder der Behandlung von Alterserscheinungen erwarten Verbraucher heutzutage zusätzliche Effekte von kosmetischen Zusammensetzungen. Ein solcher Effekt ist beispielsweise die Tönung bzw. temporäre Farbveränderung der Haut, die nach Möglichkeit nicht okklusiver Natur sein soll, wie es beispielsweise bei abdeckenden Schminken der Fall ist.

So wird zum Beispiel eine gebräunte Haut in der heutigen Zeit vom Verbraucher als Zeichen von Gesundheit und einem aktiven, sportlichen Lebensstil wahrgenommen. Eine braune Haut gilt als attraktiv und der Besitz einer leichten Hautbräune als äußerst erstrebenswert. Seit der im Jahre 1970 erstmalig beobachteten Abnahme der Ozonschicht wird jedoch auch dem Verbraucher immer mehr bewusst, dass der durch intensives Sonnenbaden hervorgerufene Bräunungsprozess mit vielfältigen Schädigungen der Haut einhergeht, welche von vorzeitiger Hautalterung bis zu einem erhöhten Hautkrebsrisiko reichen können. Mit der Anwendung von Bräunungscremes kann der Verbraucher sich den Wunsch nach einer gebräunten Haut erfüllen, ohne sich gleichzeitig verstärkt dem schädlichen UV-A und UV-B Anteil des Sonnenlichtes aussetzen zu müssen.

Die auf dem Markt befindlichen Bräunungscremes enthalten in den meisten Fällen den Wirkstoff 1,3-Dihydroxyaceton. Der Einsatz von dihydroxyacetonhaltigen Cremes kann für den Verbraucher jedoch mit Nachteilen verbunden sein. Einige Konsumenten reagieren auf Dihydroxyaceton allergisch. Der bestehende Verdacht, dass Dihydroxyaceton im Laufe der Zeit Formaldehyd abspalten könnte, lässt den Einsatz von Dihydroxyaceton in Kosmetika ebenfalls als wenig vorteilhaft erscheinen. Weiterhin hat Dihydroxyaceton den Nachteil, dass es zu Geruchsbelästigungen und zu einer ungleichmäßigen Anfärbung der Haut führen kann.

Zudem ist die Hautbräunung mit Substanzen, welche die Haut nachhaltig färben, für einige Verbraucher unerwünscht, da sie nur einzelne Bereiche ihrer Haut oder nur temporär die Hautfarbe variieren möchten.

Cremes, die mit (später leicht abwaschbaren) Pigmenten tönen, sind im Stand der Technik bekannt und erfreuen sich großer Beliebtheit als "leichtes make-up", welches gleichzeitig pflegt und nicht okklusiv wirkt. Allerdings wird das optische Erscheinungsbild dieser Cremes durch die in ihnen enthaltenen Pigmente geprägt, und manche Verbraucher lehnen es ab, ein beispielsweise hell- bis dunkelbraunes Produkt aufzutragen.

Zur Lösung dieses Problems sind Pigment- bzw. Farbstoffzubereitungen entwickelt worden, welche nach außen hell bis weiß wirken, im Inneren aber farbige Pigmente und/oder Farbstoffe beinhalten. Auf diese Weise lassen sich "magische" Cremes herstellen, die als Produkt hell bis weiß aussehen, beim Verstreichen während der Applikation aber eine sichtbare Tönung der Haut bewirken. Entsprechende Pigment- bzw. Farbstoffzubereitungen, oft als sogenannte Kapseln konfektioniert, sind im Markt breit verfügbar.

Die US 2009/155322 A1 offenbart "beads", welche eine pigmenthaltige Matrix enthalten, auf ein Coating aufgebracht worden ist, um ggf. auftretende Zersetzungsprodukte für den Konsumenten unsichtbar zu machen. In der US 2009/155321 A1 werden ähnliche "beads" offenbart.

Die WO 2004/055119 A1 offenbart Interferenzpigmente auf Basis von beschichteten plättchenförmigen Substraten.

Die DE 10 2009 060 148 A1 offenbart kosmetische Emulsionen mit einem Gehalt an Bunt- und Perlglanzpigmenten.

Ein Problem der Tönungscremes ist bislang unzureichend gelöst worden: Bei nicht vollständig homogener Applikation kann sich ein "fleckiges" Hautbild ergeben, in dem einige Bereiche intensiver gefärbt sind als andere, zudem ist ein schnelles Einziehen der Creme wichtig, um eine abriebfeste Tönung zu erreichen.

Aufgabe der vorliegenden Erfindung ist es daher, hautpflegende Mittel bereitzustellen, welche die Haut in einem schönen, gleichmäßigen Farbton zu färben vermögen, ohne das Risiko "fleckiger" Tönungen eingehen zu müssen. Darüber hinaus sollte die Abriebfestigkeit der Hauttönung "schmusefest" sein, um Transfer von Farbe z.B. bei Umarmungen zu vermeiden.

Es wurde nun gefunden, dass sich bestimmte Pigmentkapseln in Wasser-in-Öl-Emulsionen mit lamellaren flüssig-kristalline Strukturen stabil einarbeiten lassen und diese speziellen Emulsionen eine hoch gleichmäßige Applikation ermöglichen, die zu einem ebenmäßigen Hautbild führt. Darüber hinaus ziehen diese Emulsionen hervorragende ein und bewirken eine schnell abriebfeste Tönung der behandelten Hautareale.

Gegenstand der vorliegenden Erfindung ist in einer ersten Ausführungsform eine Öl-in-Wasser-Emulsion, enthaltend - bezogen auf ihr Gewicht -
a) mindestens 40 Gew.-% Wasser,
b) mindestens 0,5 Gew.-% mindestens eines primären, linearen, gesättigten C₁₄₋₃₀-Fettalkohols,
c) mindestens eine mehrschichtige Pigmentkapsel, umfassend
   i) einen Partikelkern,
   ii) eine diesen Partikelkern umgebende erste Hülle, welche - bezogen auf ihr Gewicht - mindestens 50 Gew.-% farbige Pigmente enthält,
   iii) eine die erste Hülle umgebende zweite Hülle, welche - bezogen auf ihr Gewicht - mindestens 50 Gew.-% Titandioxid enthält,
   iv) eine die zweite Hülle umgebende dritte Hülle aus einem wasserunlöslichen, thermoplastischem Polymer,
dadurch gekennzeichnet, dass
- die Emulsion lamellare flüssig-kristalline Strukturen aufweist und
- der Partikelkern der mehrschichtigen Pigmentkapsel
   - 20 bis 40 Gew.-% des Gesamt-Kapselgewichts ausmacht und
   - zu mindestens 50 Gew.-% seines Gewichts aus Alditolen, vorzugsweise aus Mannitol, besteht.

Die erfindungsgemäßen Öl-in-Wasser-Emulsionen können als innere Phase entweder eine kosmetische Öl- oder Fettkomponente oder eine Wasser-in-Öl-Emulsion enthalten. Im letzteren Fall stellen die erfindungsgemäßen Lamellaremulsionen Wasser-in-Öl-in-Wasser-Emulsionen dar.

Die erfindungsgemäßen Zusammensetzungen enthalten mindestens 0,5 Gew.-% mindestens eines primären, linearen, gesättigten C₁₄₋₃₀-Fettalkohols.
Bevorzugte erfindungsgemäße lamellare Öl-in-Wasser-Emulsionen sind dadurch gekennzeichnet, dass mindestens ein primärer, linearer, gesättigter Fettalkohol mit einer Kettenlänge von 14-30, bevorzugt 20 - 40 Kohlenstoffatomen enthalten ist.
Besonders bevorzugte erfindungsgemäße lamellare Öl-in-Wasser-Emulsionen sind dadurch gekennzeichnet, dass sie Behenylalkohol und/oder Arachidylalkohol, insbesondere als technische Mischung beider Alkohole, enthalten. Die auf diesen längerkettigen Fettalkoholen basierenden lamellaren Phasen sind weniger störanfällig gegenüber anderen Bestandteilen der Emulsion.
Besonders bevorzugte erfindungsgemäße Zusammensetzungen enthalten mindestens einen primären, linearen, gesättigten Fettalkohol mit einer Kettenlänge von 14 - 30, bevorzugt 20 - 40 Kohlenstoffatomen in einer Gesamtmenge von 0,5 -15 Gew.-%, bevorzugt 0,8-10 Gew.-%, besonders bevorzugt 1 - 8 Gew.-% und außerordentlich bevorzugt 1,5 - 5 Gew.-%, jeweils bezogen auf das Gewicht der gesamten Zusammensetzung.

Bevorzugte lineare primäre Fett- oder Wachsalkohole mit einer Kettenlänge der Alkylgruppe von 20 - 40 Kohlenstoffatomen sind ausgewählt aus Arachidylalkohol (1-Eicosanol), Behenylalkohol, Lignocerylalkohol (1-Tetracosanol, C₂₄H₅₀O), Cerylalkohol (C₂₆H₅₄O), Myricylalkohol (1-Triacontanol, C₃₀H₆₂O) und Melissylalkohol (C₃₁H₆₄O, 1-Hentriacontanol). Besonders bevorzugt sind Arachidylalkohol und Behenylalkohol. In einer bevorzugten Ausführungsform der Erfindung ist Arachidylalkohol in Mengen von 0,05 - 5 Gew.-%, besonders bevorzugt 0,1 - 4 Gew.-%, jeweils bezogen auf das Gewicht der gesamten Zusammensetzung, enthalten. In einer weiteren bevorzugten Ausführungsform der Erfindung ist Behenylalkohol in Mengen von 0,5 - 4 Gew.-%, besonders bevorzugt 1 - 2 Gew.-%, jeweils bezogen auf das Gewicht der gesamten Zusammensetzung, enthalten. In einer weiteren besonders bevorzugten Ausführungsform der Erfindung ist eine Mischung aus Arachidylalkohol und Behenylalkohol enthalten, wobei die für die Einzelsubstanzen genannten bevorzugten Mengenangaben auch für die Mischung als bevorzugt gelten.

Ganz besonders bevorzugte erfindungsgemäße lamellare Öl-in-Wasser-Emulsionen sind dadurch gekennzeichnet, dass sie mindestens einen Alkohol, ausgewählt aus Behenylalkohol und Arachidylalkohol sowie Mischungen hiervon, in einer Gesamtmenge von 0,5 bis 10 Gew.-%, vorzugsweise 0,6 bis 9 Gew.-%, weiter bevorzugt 0,7 bis 8 Gew.-%, noch weiter bevorzugt 0,8 bis 7 Gew.-%, besonders bevorzugt 0,9 bis 6 Gew.-% und insbesondere 1 bis 5 Gew.-%, jeweils bezogen auf das Gewicht der Emulsion, enthalten.

Die Ausbildung lamellarer Flüssigkristallphasen kann durch die Anwesenheit von partiell oder ganz neutralisierten C₆ - C₃₀-Fettsäuren behindert oder sogar unterdrückt werden. Besonders bevorzugte erfindungsgemäße lamellare Öl-in-Wasser-Emulsionen sind daher dadurch gekennzeichnet, dass sie von partiell oder ganz neutralisierten C₆ - C₃₀-Fettsäuren frei sind.

Als hydrophile Emulgatoren zur Herstellung der erfindungsgemäßen Öl-in-Wasser-Emulsionen eignen sich alle für die Emulgierung kosmetischer Öl- und Fettkomponenten geeigneten Tenside. Dies sind vor allem ionische Emulgatoren oder nichtionische Emulgatoren mit einem HLB-Wert von 8 bis 18. Der HLB-Wert stellt dabei einen Wert dar, der sich aus der Struktur des Moleküls nach der Formel HLB = 0,2 x (100 - L) ergibt, worin L der prozentuale Gewichtsanteil der lipophilen Alkyl-, Alkenyl- oder Acylgruppen im Molekül ist.

Als ionische Emulgatoren eignen sich anionische, kationische, zwitterionische und amphotere Tenside, bevorzugt solche mit einer primären, linearen Alkyl- oder Alkenylgruppe mit 12 - 18 C-Atomen. Geeignete anionische Emulgatoren sind z.B. die Salze von C₁₂-C₁₈-Fettsäuren, von Schwefelsäuremonoestern oder Phosphorsäure-monoestern von C₁₂-C₁₈-Fettalkoholen, von C₁₂-C₁₈-Acylisethionsäuren, von C₁₂-C₁₈-Alkansulfonsäuren oder von C₁₂-C₁₈-Acylaminosäuren. Kationische Emulgatoren sind z.B. Cetyl-trimethylammonium-chlorid oder Distearoxyethyl-hydroxyethyl-methylammonium-chlorid. Geeignete zwitterionische Tenside sind z.B. Betaintenside wie Stearamidopropyldimethyl-carboxymethylammonium-betain und geeignete Amphotensi-de sind z.B. Cetylaminopropionsäure oder Kokoamphocarboxyglycinat. Auch Amin-oxidtenside eignen sich als hydrophile Emulgatoren.

Geeignete nichtionogene Tenside mit HLB-Werten von 8 bis 18 sind insbesondere die Anlagerungsprodukte von Ethylenoxid an Fettsäuren, an Fettalkohole, an Fettsäurealkanolamide, an Fettsäuremonoglyceride, an Sorbitanfettsäureester, an Methylglucosid-Fettsäureester oder an andere Lipide mit Carboxyl-, Hydroxyl- oder Aminogruppen, wobei der Anteil der dabei gebildeten Ethoxygruppen wenigstens 40 Gew.-% ausmachen sollte. Weitere geeignete nichtionische Tenside sind Alkylpolyglucoside, Zuckerester und Polyglycerin-Fettsäureester.

Erfindungsgemäß bevorzugte Emulsionen enthalten mindestens ein Salz eines C₁₂₋₂₀-Alkylphosphats, insbesondere ein Salz von Cetylphosphat, als anionischen Öl-in-Wasser-Emulgator.

Erfindungsgemäß bevorzugte Salze von C₁₂₋₂₀-Alkylphosphaten sind ausgewählt aus den Monoestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt als Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäuremonoester. Besonders bevorzugt ist Dikaliummonocetylphosphat.
Weitere erfindungsgemäß bevorzugte Salze von C₁₂₋₂₀-Alkylphosphaten, die als anionischer ÖI-in-Wasser-Emulgator einen ersten Teil des erfindungsgemäßen O/W-Emulgatorsystems bilden, sind ausgewählt aus den Diestern von Phosphorsäure mit Laurylalkohol, Tridecylalkohol, Isotridecylalkohol, Myristylalkohol, Pentadecylalkohol, Cetylalkohol, Palmitylalkohol, Isocetylalkohol, Isostearylalkohol, Stearylalkohol, Oleylalkohol, Elaidylalkohol, Linoleylalkohol, Linolenylalkohol, Nonadecylalkohol, Arachylalkohol, Gadoleylalkohol oder Arachidonalkohol, die als Alkali-, Erdalkali-, Ammonium-, Alkylammonium-, Alkanolamin- oder Glucammoniumsalz, bevorzugt als Natrium-, Kalium-, Alkanolamin-, Trialkylammonium-, Triethanolamin-, 2-Amino-1-butanol-, 2-Amino-2-methyl-1-propanol-, 2-Amino-2-methyl-1,3-propandiol-, 2-Amino-2-ethyl-1,3-propandiol- oder Tris-(hydroxymethyl)aminomethan-Salze sowie als Salze der basischen Aminosäuren Ornithin, Lysin, Arginin und/oder Histidin, vorliegen. Bevorzugt sind die Kaliumsalze der genannten Phosphorsäurediester. Besonders bevorzugt ist Kaliumdicetylphosphat.
Besonders bevorzugt sind Mischungen aus Mono-C₁₂₋₂₀-Alkylphosphaten und Di-C₁₂₋₂₀-Alkylphosphaten. Außerordentlich bevorzugt sind Mischungen aus Dikaliummonocetylphosphat und Kaliumdicetylphosphat.
Das mindestens eine Salz eines C₁₂₋₂₀-Alkylphosphats ist in den bevorzugten erfindungsgemäßen Zusammensetzungen vorzugsweise in einer Gesamtmenge von 0,5 - 2,0 Gew.-%, bevorzugt 0,8 - 1,8 Gew.-%, besonders bevorzugt 1,2 - 1,5 Gew.-%, jeweils bezogen auf das Gesamtgewicht der Zusammensetzung, enthalten. Bevorzugte erfindungsgemäße lamellare Öl-in-Wasser-Emulsionen enthalten mindestens ein Salz von C₁₂₋₂₀-Alkylphosphaten, insbesondere mindestens ein Salz von Cetylphosphat, in einer Gesamtmenge von 0,5 bis 10 Gew.-%, vorzugsweise 0,6 bis 9 Gew.-%, weiter bevorzugt 0,7 bis 8 Gew.-%, noch weiter bevorzugt 0,8 bis 7 Gew.-%, besonders bevorzugt 0,9 bis 6 Gew.-% und insbesondere 1 bis 5 Gew.-%, jeweils bezogen auf das Gewicht der Emulsion.

Erfindungsgemäß kann ein O/W-Emulgatorsystem eingesetzt werden, das zusätzlich C₁₄₋₂₀-Mono- oder Diacylglyceride enthält. Diese sind vorzugsweise ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon. Weitere erfindungsgemäß bevorzugte C₁₄₋₂₀-Mono- oder Diacylglyceridmischungen sind Glyceride gehärteter, das heißt, hydrierter, bevorzugt vollständig hydrierter, Fettsäuren natürlicher Öle. Erfindungsgemäß besonders bevorzugt sind gehärtete Palmölglyceride.
Das mindestens eine C₁₄₋₂₀-Mono- oder Diacylglycerid ist in solchen bevorzugten erfindungsgemäßen Zusammensetzungen in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung, enthalten.
Bevorzugte erfindungsgemäße Zusammensetzungen enthalten mindestens eine Substanz, ausgewählt aus Monomyristoylglycerid, Monopalmitoylglycerid, Monostearoylglycerid, Monoarachinoylglycerid, Dimyristoylglycerid, Dipalmitoylglycerid, Distearoylglycerid und Diarachinoylglycerid sowie aus Mischungen hiervon, in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung.
Weitere bevorzugte erfindungsgemäße Zusammensetzungen enthalten als C₁₄₋₂₀-Mono- oder Diacylglyceridmischung gehärtete Palmölglyceride in einer Gesamtmenge von 0,3 - 1,7 Gew.-%, bevorzugt 0,6 - 1,4 Gew.-%, besonders bevorzugt 0,9 - 1,1 Gew.-%, jeweils bezogen auf das Gesamtgewicht der Zusammensetzung.
Weitere bevorzugte erfindungsgemäße Zusammensetzungen enthalten mindestens ein Salz von C₁₂₋₂₀-Alkylphosphaten und mindestens ein C₁₄₋₂₀-Mono- oder Diacylglycerid im Gewichtsverhältnis 2/3 bis 3/2, bevorzugt 1/1 bis 3/2, besonders bevorzugt 6/5 bis 3/2.

Eine erfindungsgemäß besonders bevorzugtes O/W-Emulgatorsystem, umfassend ein Gemisch aus Dikaliummonocetylphosphat und Kaliumdicetylphosphat mit gehärteten Palmölglyceriden, ist als Handelsprodukt "Emulsiphos 677660" (INCI: Potassium Cetyl Phosphate, Hydrogenated Palm Glycerides) von der Firma Symrise erhältlich.

Als Öl- und Fettkomponenten eignen sich alle pflanzlichen, tierischen, mineralischen und synthetischen Öle, Fette und Wachse, die für eine Anwendung am menschlichen Körper aus physiologischen und ästhetischen Gründen infrage kommen. Geeignet sind z.B. Paraffine, Fettsäureester einwertiger oder mehrwertiger Alkohole, z.B. Triglyceride, Fettsäure-Fettalkohol-Ester, Fettsäure-Dicarbonsäure-Polyol-Polyester, Fettalkohol-Diol-Dicarbonsäure-Polyester, Di-n-alkylether, Polyolefine oder Silikonöle. Bevorzugt werden flüssige Öle oder Gemische aus Ölen und Wachsen eingesetzt, die bei 20°C flüssig sind. Als Ölkomponenten geeignete Monoester sind z.B. die Methylester und Isopropylester von Fettsäuren mit 12 - 22 C-Atomen, wie z.B. Methyllaurat, Methylstearat, Methyloleat, Methylerucat, Isopropylpalmitat, Isopropylmyristat, Isopropylpalmitat, Isopropylstearat, Isopropyloleat. Andere geeignete Monoester sind z.B. n-Butylstearat, n-Hexyllaurat, n-Decyloleat, Isooctylstearat, Isononylpalmitat, Isononyl-isononanoat, 2-Ethylhexyl-palmitat, 2-Ethylhexyl-laurat, 2-Hexyldecyl-stearat, 2-Octyldodecyl-palmitat, Oleyloleat, Oleylerucat, Erucyloleat sowie Ester, die aus technischen aliphatischen Alkoholgemischen und technischen aliphatischen Carbonsäuren erhältlich sind, z.B. Ester aus gesättigten und ungesättigten Fettalkoholen mit 12 - 22 C-Atomen und gesättigten und ungesättigten Fettsäuren mit 12 - 22 C-Atomen, wie sie aus tierischen und pflanzlichen Fetten zugänglich sind. Geeignet sind auch natürlich vorkommende Monoester- bzw. Wachsester-Gemische, wie sie z.B. im Jojobaöl oder im Spermöl vorliegen.

Geeignete Dicarbonsäureester sind z.B. Di-n-butyl-adipat, Di-n-butyl-sebacat, Di-(2-ethylhexyl)-adipat, Di-(2-hexyldecyl)-succinat und Di-isotridecyl-acelaat. Geeignete Diolester (III) sind z.B. Ethylenglycoldioleat, Ethylenglycol-di-isotridecanoat, Propylen-glycol-di-(2-ethylhexanoat), Butandiol-di-isostearat und Neopentylglycol-di-caprylat.

Als Fettsäuretriglyceride können natürliche, pflanzliche Öle, z.B. Olivenöl, Sonnenblumenöl, Sojaöl, Erdnußöl, Rapsöl, Mandelöl, Palmöl, aber auch die flüssigen Anteile des Kokosöls oder des Palmkernöls sowie tierische Öle, wie z.B. Klauenöl, die flüssigen Anteile des Rindertalges oder auch synthetische Triglyceride, wie sie durch Veresterung von Glycerin mit Fettsäuren mit 8 - 22 C-Atomen erhalten werden, z.B. Triglyceride von Caprylsäure-Caprinsäure-Gemischen, Triglyceride aus technischer Ölsäure oder aus Palmitinsäure-Gemischen eingesetzt werden.

Als Ölkomponente können die erfindungsgemäßen Zusammensetzungen n-Hexadecyl-n-nonanoat, n-Octadecyl-n-nonanoat oder eine Mischung hiervon in einer Gesamtmenge von 0,7 - 2 Gew.-%, bezogen auf das Gesamtgewicht der Zusammensetzung enthalten. Überraschend wurde festgestellt, dass sich mit diesen Estern in einer Gesamtmenge von 0,7 - 2 Gew.-%, bezogen auf das Gesamtgewicht der Zusammensetzung, Probleme bei der Herstellung lagerstabiler Pigmentpartikel-haltiger Emulsionen erfolgreich lösen ließen. Bevorzugte erfindungsgemäße Zusammensetzungen enthalten n-Hexadecyl-n-nonanoat, n-Octadecyl-n-nonanoat oder eine Mischung hiervon in einer Gesamtmenge von 1 - 1,5 Gew.-%, bezogen auf ihr Gesamtgewicht. Weitere bevorzugte erfindungsgemäße Zusammensetzungen enthalten, bezogen auf ihr Gesamtgewicht, eine Mischung von n-Hexadecyl-n-nonanoat und n-Octadecyl-n-nonanoat in einer Gesamtmenge von 0,7 - 2 Gew.-%, bevorzugt 1 - 1,5 Gew.-%.

Als weitere Ölkomponente können die erfindungsgemäßen Zusammensetzungen, bezogen auf ihr Gesamtgewicht, 2 - 6 Gew.-%, bevorzugt 2,5 - 5 Gew.-%, besonders bevorzugt 3 - 4 Gew.-%, 2-Ethylhexyl-3,5,5-trimethylhexanoat enthalten. Überraschend wurde festgestellt, dass sich mit diesem Öl in der vorstehend angegebenen Menge in Kombination mit n-Hexadecyl-n-nonanoat, n-Octadecyl-n-nonanoat oder einer Mischung hiervon in einer Gesamtmenge von 0,7 - 2 Gew.-% Probleme bei der Herstellung lagerstabiler Pigmentpartikel-haltiger Emulsionen erfolgreich lösen ließen.
"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die Anwesenheit lamellarer Flüssigkristallphasen schließt aus, dass es sich bei den erfindungsgemäßen lamellaren Öl-in-Wasser-Emulsionen um Mikroemulsionen oder PIT-Emulsionen handelt. Besonders bevorzugte erfindungsgemäße lamellare Öl-in-Wasser-Emulsionen sind daher dadurch gekennzeichnet, dass sie keine Mikroemulsion und/oder keine PIT-Emulsion sind.

Die erfindungsgemäßen Zusammensetzungen enthalten mindestens eine mehrschichtige Pigmentkapsel, die einen Partikelkern, eine diesen Partikelkern umgebende erste Hülle, welche - bezogen auf ihr Gewicht - mindestens 50 Gew.-% farbige Pigmente enthält, eine die erste Hülle umgebende zweite Hülle, welche - bezogen auf ihr Gewicht - mindestens 50 Gew.-% Titandioxid enthält und eine die zweite Hülle umgebende dritte Hülle aus einem wasserunlöslichen, thermoplastischem Polymer umfasst. Nach außen ist diese Kapsel durch das wasserunlösliche, thermoplastische Polymer abgeschlossen und kann sowohl in wässrigen als auch in ölbasierten kosmetischen Systemen eingesetzt werden, ohne dass ein Ausbluten oder eine mangelnde Lagerstabilität zu befürchten wäre. Erst durch mechanische Einwirkung bei der Applikation des Kosmetikums "zerbricht" die Kapsel und setzt die Pigmente aus den inneren Hüllschichten frei.

Das optische Erscheinungsbild der Kapseln wird durch die unter der äußeren Hülle befindliche zweite Hülle geprägt. Durch den hohen Gehalt an Titandioxid in dieser Hülle wirken die Kapseln nach außen hell bis weiß, auch wenn in der ersten, direkt auf dem Partikelkern befindlichen Schicht dunkle Pigmente enthalten sind.

Das Kapselinnere wird durch einen Partikelkern gebildet. Als Partikelkern kommen grundsätzlich alle bei Raumtemperatur (25°C) festen Substanzen in Frage, im Hinblick auf die rückstandsfreie Applikation sind wasserlösliche Substanzen bevorzugt. Besonders bevorzugte Trägermaterialien sind ausgewählt aus Zuckern und Zuckeralkoholen, unter diesen sind die Zuckeralkohole besonders bevorzugt. Besonders bevorzugte Alditole sind Mannit (Mannitol), Isomalt, Lactit, Sorbit (Sorbitol oder Glucitol) und Xylit (Xylitol), Threit, Erythrit und Arabit.

Erfindungsgemäße lamellare Öl-in-Wasser-Emulsionen sind dadurch gekennzeichnet, daß der Partikelkern der mehrschichtigen Pigmentkapsel 20 bis 40 Gew.-% des Gesamt-Kapselgewichts ausmacht und zu mindestens 50 Gew.-% seines Gewichts aus Alditolen, vorzugsweise aus Mannitol, besteht.
In bevorzugten erfindungsgemäßen lamellaren Öl-in-Wasser-Emulsionen besteht der Partikelkern zu mindestens 60 Gew.-%, weiter bevorzugt zu mindestens 70 Gew.-% und insbesondere zu mindestens 75 Gew.-% seines Gewichts aus Alditolen, vorzugsweise aus Mannitol.

Ganz besonders bevorzugte erfindungsgemäße lamellare Öl-in-Wasser-Emulsionen enthalten mehrschichtigen Pigmentkapseln, welche - bezogen auf das Gewicht der Pigmentkapsel - 10 bis 40 Gew.-%, vorzugsweise 12,5 bis 37,5 Gew.-%, weiter bevorzugt 15 bis 35 Gew.-%, noch weiter bevorzugt 17,5 bis 32,5 Gew.-%, besonders bevorzugt 20 bis 30 Gew.-% und insbesondere 22,5 bis 27,5 Gew.-% Mannitol enthalten.

Die erfindungsgemäßen Zusammensetzungen ergeben bei Applikation überraschende Effekte, indem eine nach außen helle bis weiße Zusammensetzung einen dunkleren Applikationserfolg herbeiführt. Dies ist die Folge des Kollabierens der mehrschichtigen Pigmentkapseln, wobei die von außen durch die dritte und zweite Hülle zunächst nicht sichtbaren farbigen Pigmente der ersten Hülle freigesetzt werden. Die den Partikelkern umgebende erste Hülle, enthält - bezogen auf ihr Gewicht - mindestens 50 Gew.-% farbige Pigmente.

Bevorzugte Pigmente dieser Art können anorganisch oder organisch sein. Weitere bevorzugte Pigmente weisen einen zahlenmittleren Teilchendurchmesser von 0,1 - 200 µm, bevorzugt 0,5 - 100 µm, besonders bevorzugt 1 - 50 µm und außerordentlich bevorzugt 2 - 30 µm auf.
Die Farbpigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Besonders bevorzugte Farbpigmente sind beispielsweise Eisenoxide (z. B. Fe₂O₃, Fe₃O₄, FeO(OH)) und/oder Zinnoxid.

Einsetzbare farbige Pigmente sind beispielsweise Pigment Green (Cl 10006, grün), Pigment Yellow 1 (Cl 11680, gelb), Pigment Yellow 3 (Cl 11710, gelb), Pigment Orange 1 (Cl 11725, orange), Pigment Red 3 (Cl 12120, rot), Pigment Red 112 (Cl 12370, rot), Pigment Red 7 (Cl 12420, rot), Pigment Brown 1 (Cl 12480, braun), Pigment Yellow 13 (Cl 21100, gelb), Pigment Yellow 83 (Cl 21108, gelb), Pigment Violet 23 (Cl 51319, violett), Pigment Red 122 (Cl 73915, rot), Pigment Blue 16 (Cl 74100, blau), Pigment Red 101 und 102 (Cl 77015, rot), Kohlenstoff (Cl 77266, schwarz), Pigment Black 9 (Cl 77267, schwarz), Carbo medicinalis vegetabilis (Cl 77268:1, schwarz), Chromoxid (Cl 77288, grün), Chromoxid, wasserhaltig (Cl 77289, grün), Pigment Blue 28, Pigment Green 14 (Cl 77346, grün), Pigment Metal 2 (Cl 77400, braun), Gold (Cl 77480, braun), Eisenoxide und -hydoxide (Cl 77489, orange), Eisenoxid (Cl 77491, rot), Eisenoxidhydrat (Cl 77492, gelb), Eisenoxid (Cl 77499, schwarz), Mischungen aus Eisen(II)- und Eisen(III)-hexacyanoferrat (Cl 77510, blau), Silber (Cl 77820, weiß).

Die Wahl der Pigmente in der ersten Hülle kann dem gewünschten Anwendungszweck des Kosmetikums angepasst werden. Um beispielsweise eine "gesunde Bräune" herbeizuführen, können gelbe, orange oder braune bis schwarze Pigmente oder deren Mischungen eingesetzt werden.

Zur Kaschierung von unerwünschten Hautrötungen werden im Sinne der Komplementärfarblehre vorteilhaft Rotlicht filternde Farbpigmente, insbesondere Grünpigmente, eingesetzt. Aber auch Mischungen können besondere Effekte bei der Teint-Korrektur ergeben. So ist der Zusatz von Blaupigmenten, bevorzugt im Gewichtsverhältnis 1:1 bis 1:100 in Bezug auf das eingesetzte Grünpigment, erfindungsgemäß bevorzugt. Das Blaupigment erhöht die farbverändernde Wirkung, so dass die rötliche Hautfarbe besser in ein natürliches Braun, d.h. eine natürliche Hautfarbe, umgewandelt wird.

Vorteilhafte Grünpigmente können aus der folgenden Gruppe allein oder in Kombination ausgewählt werden: Colorona Brilliant Green, Colorona Egyptian Emerald, Timiron Splendid Green, Vert Oxyde Crome Anhydre N, Vert Oxyde Crome Hydrate W886 und/oder Outremer Supercosmetique W 6803. Diese genannten Pigmente werden u. a. auch unter den entsprechenden Cl Nummern gelistet. Bevorzugt sind daher insbesondere die Grünpigmente mit den Cl Nummern Cl 77288, Cl 77289, Cl 77007, Cl 77891, Cl 77491, Cl 77499, Cl 77891, Cl 77499, Cl 77288 und Cl 77492.

Rotlicht filternd bedeutet hierin, dass der Rotlichtanteil der sichtbaren Lichtes absorbiert wird und die Komplementärfarbe Grün sichtbar wird.

Durch den Auftrag einer erfindungsgemäßen Zubereitung mit Rotlicht filternden Farbstoffen, wie beispielsweise die angegebenen Grünpigmente, auf die Haut, die beispielsweise durch Sonnenbestrahlung oder aufgrund Rosacea rötlich gefärbt erscheint, wird für den Betrachter das Erscheinungsbild der Haut natürlich erscheinen. Um diesen Effekt zu generieren, ist die Auswahl und Menge an Rotlicht filternden Farbstoffen wesentlich.

Erfindungsgemäß bevorzugte lamellare Öl-in-Wasser-Emulsionen sind dadurch gekennzeichnet, daß die den Partikelkern umgebende erste Hülle
- 20 bis 40 Gew.-% des Gesamt-Kapselgewichts ausmacht und
- zu mindestens 50 Gew.-%, vorzugsweise zu mindestens 60 Gew.-%, weiter bevorzugt zu mindestens 70 Gew.-% und insbesondere zu mindestens 75 Gew.-% ihres Gewichts aus einem oder mehreren der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz) besteht.

Besonders bevorzugte erfindungsgemäße lamellare Öl-in-Wasser-Emulsionen enthalten mehrschichtigen Pigmentkapseln, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und - hydoxide (Cl 77489 orange), Eisenoxid (Cl 77491 rot), Eisenoxid (Cl 77499 schwarz) enthalten.

Ganz besonders bevorzugte erfindungsgemäße lamellare Öl-in-Wasser-Emulsionen enthalten mehrschichtigen Pigmentkapseln, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz)
enthalten.

Um die überraschenden Effekte bei der Applikation zu gewährleisten, wird in den Pigmentkapseln in den erfindungsgemäßen Zubereitungen die farbige erste Hülle durch eine weiße zweite Hülle "kaschiert", welche - bezogen auf ihr Gewicht - mindestens 50 Gew.-% Titandioxid enthält.

Erfindungsgemäß bevorzugte lamellare Öl-in-Wasser-Emulsionen sind dadurch gekennzeichnet, dass die die erste Hülle umgebende zweite Hülle
- 20 bis 40 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Titandioxid (Cl 77891) besteht.

Besonders bevorzugte erfindungsgemäße lamellare Öl-in-Wasser-Emulsionen enthalten mehrschichtigen Pigmentkapseln, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% Titandioxid (Cl 77891).

Ganz besonders bevorzugte erfindungsgemäße lamellare Öl-in-Wasser-Emulsionen enthalten mehrschichtigen Pigmentkapseln, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz) und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% Titandioxid (Cl 77891)
enthalten.

Nach außen werden die in den erfindungsgemäßen Zusammensetzungen eingesetzten Pigmentkapseln durch eine dritte Hülle aus einem wasserunlöslichen, thermoplastischem Polymer abgeschlossen. Unter "Polymer" werden dabei im Rahmen der vorliegenden Erfindung sowohl Homoals auch Copolymere als auch Polymergemische verstanden.

Zu den erfindungsgemäß geeigneten Thermoplasten zählen z. B.: Acrylnitril-Butadien-Styrol (ABS), Polyamide (PA), Polylactat (PLA), Polymethylmethacrylat (PMMA), Polycarbonat (PC), Polyethylenterephthalat (PET), Polyethylen (PE), Polypropylen (PP), Polystyrol (PS), Polyetheretherketon (PEEK) und Polyvinylchlorid (PVC).

Im Hinblick auf die optischen Eigenschaften der Pigmentkapseln, auf ihre Verarbeitbarkeit (Herstellung der Kapseln, statische Aufladung und Stauben bei der Verarbeitung etc.) sowie auf ihre Verträglichkeit mit den übrigen Bestandteilen der erfindungsgemäßen Zusammensetzungen hat sich insbesondere Polymethylmethacrylat (PMMA) als besonders geeignetes Material für die dritte Hülle erwiesen.

Erfindungsgemäß bevorzugte lamellare Öl-in-Wasser-Emulsionen sind dadurch gekennzeichnet, dass die die zweite Hülle umgebende dritte Hülle
- 2 bis 7 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Polymethylmethacrylat besteht.

Besonders bevorzugte erfindungsgemäße lamellare Öl-in-Wasser-Emulsionen enthalten mehrschichtigen Pigmentkapseln, welche - bezogen auf das Gewicht der Pigmentkapsel - 1 bis 7 Gew.-%, vorzugsweise 2 bis 6,75 Gew.-%, weiter bevorzugt 2,5 bis 6,5 Gew.-%, noch weiter bevorzugt 3 bis 6,25 Gew.-%, besonders bevorzugt 3,5 bis 6 Gew.-% und insbesondere 4 bis 5,75 Gew.-% Polymethylmethacrylat (PMMA).

Ganz besonders bevorzugte erfindungsgemäße lamellare Öl-in-Wasser-Emulsionen enthalten mehrschichtigen Pigmentkapseln, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz) und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% Titandioxid (Cl 77891) und
- 1 bis 7 Gew.-%, vorzugsweise 2 bis 6,75 Gew.-%, weiter bevorzugt 2,5 bis 6,5 Gew.-%, noch weiter bevorzugt 3 bis 6,25 Gew.-%, besonders bevorzugt 3,5 bis 6 Gew.-% und insbesondere 4 bis 5,75 Gew.-% Polymethylmethacrylat (PMMA)
enthalten.

Die in den erfindungsgemäßen Zusammensetzungen eingesetzten Pigmentkapseln können beliebige Partikelgrößen aufweisen. Größere Kapseln mit Durchmessern bis zu 2 mm können optisch reizvolle Akzente in der Formulierung setzen, haben aber den Nachteil, die farbigen Pigmente bei der Applikation lokal in größerer Menge freizusetzen, so dass für ein einheitliches Anwendungsergebnis ein umfangreicheres Verreiben erforderlich ist. Kleinere Kapseln können in größerer Menge eingesetzt werden und erzielen so bereits mit wenig Applikationsaufwand eine einheitliche und homogene Freisetzung der verkapselten Farbpigmente.

Besonders bevorzugte erfindungsgemäße lamellare Öl-in-Wasser-Emulsionen sind dadurch gekennzeichnet, dass mindestens 90 Gew.-% der mehrschichtigen Pigmentkapseln eine Partikelgröße von 75 nm bis 211 µm (70 bis 200 Mesh) aufweisen.

Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, anfeuchtende und/oder feuchthaltende Substanzen, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft, Vitamine oder Antifalten-Wirkstoffe.
Vorteilhafte Konservierungsmittel sind beispielsweise lodopropylbutylcarbamate, Parabene, Phenoxyethanol, Ethanol, Benzoesäure und andere. Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.
Besonders bevorzugte Zusammensetzungen werden ferner erhalten, wenn als Zusatz- oder Wirkstoffe Antioxidantien eingesetzt werden. Erfindungsgemäß bevorzugt enthalten die Zusammensetzungen mindestens ein Antioxidans. Als günstige, aber dennoch fakultativ zu verwendende Antioxidantien können alle für kosmetische und/oder dermatologische Anwendungen geeigneten oder gebräuchlichen Antioxidantien verwendet werden, insbesondere Tocopherol, Tocopherylacetat oder Dimethylmethoxy Chromanol, erhältlich unter dem Handelsnamen Lipochroman-6 von Lipotec.
Weiter vorteilhaft werden der oder die Wirkstoffe gewählt aus der Gruppe, die Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).
Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R, 3R)-3,3',4',5,7-Flavanpentaol) ist ein vorteilhafter Wirkstoff im Sinne der vorliegenden Erfindung.
In einer weiteren bevorzugten Ausführungsform enthalten die erfindungsgemäßen Zusammensetzungen mindestens ein Flavonoid oder mindestens einen Flavonoid-reichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-a-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Zusammensetzungen enthalten mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 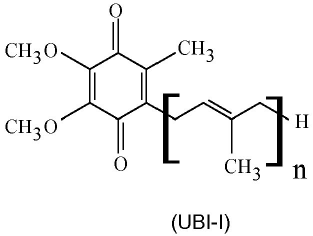 mit n = 6, 7, 8, 9 oder 10.
Besonders bevorzugt ist das Ubichinon der Formel (UBI-I) mit n = 10, auch bekannt als Coenzym Q10.

Erfindungsgemäß besonders bevorzugte Zusammensetzungen enthalten mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Die Herkunft der eingesetzten natürlichen Betainverbindung kann erfindungsgemäß durchaus synthetisch sein. Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻), Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II) 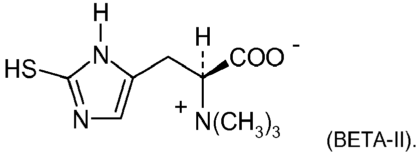 Weiterhin können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung zu verwenden. Es kann auch von Vorteil sein, beliebige Enantiomerengemische, beispielsweise ein Racemat aus D-und L-Form, zu verwenden. Bevorzugte erfindungsgemäße Zusammensetzungen sind dadurch gekennzeichnet, dass sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Die erfindungsgemäßen kosmetischen oder dermatologischen Zusammensetzungen liegen in Form einer flüssigen, fließfähigen oder festen Öl-in-Wasser-Emulsion vor.
Weitere bevorzugte Zusatzstoffe sind Verdickungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolamin-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren, wobei Acryloyldimethyltaurat ein besonders bevorzugtes Sulfonsäuregruppen-haltiges Monomer darstellt. Diese Copolymere können bevorzugt vernetzt vorliegen. Geeignete Handelsprodukte sind Sepigel^{®}305, Simulgel^{®} 600, Simulgel^{®} NS, Simulgel^{®} EPG und Simulgel^{®} EG von SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2-20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex Noveon/Lubrizol).
Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.
Bevorzugte erfindungsgemäße Sonnenschutz-Zusammensetzungen enthalten zur Verdickung der wässrigen Phase und damit zur zusätzlichen Stabilisierung der gesamten Zusammensetzung eine Mischung aus Xanthan-Gum, mindestens einem vernetzten Polyacrylat und mindestens einem vernetzten Acryloyldimethyltaurat-Copolymer. Besonders bevorzugt sind Mischungen aus Xanthan-Gum, mindestens einem vernetzten Polyacrylat und mindestens einem vernetzten Acryloyldimethyltaurat-Copolymer in einer Gesamtmenge von 0,5 - 1,5 Gew.-%, bevorzugt 0,7 - 1,3 Gew.-% und außerordentlich bevorzugt 1 - 1,2 Gew.-%, jeweils bezogen auf das Gesamtgewicht der Zusammensetzung.

Gegenstand der Erfindung ist ferner die Verwendung von mehrschichtigen Pigmentkapseln, umfassend
i) einen Partikelkern,
ii) eine diesen Partikelkern umgebende erste Hülle, welche - bezogen auf ihr Gewicht - mindestens 50 Gew.-% farbige Pigmente enthält,
iii) eine die erste Hülle umgebende zweite Hülle, welche - bezogen auf ihr Gewicht - mindestens 50 Gew.-% Titandioxid enthält,
iv) eine die zweite Hülle umgebende dritte Hülle aus einem wasserunlöslichen, thermoplastischem Polymer,
in O/W-Emulsionen mit lamellaren flüssig-kristallinen Strukturen.

Bezüglich weiterer bevorzugter Ausführungsformen der erfindungsgemäßen Verwendungen gilt mutatis mutandis das zu den erfindungsgemäßen Zusammensetzungen Gesagte.

## Patentansprüche

1. Öl-in-Wasser-Emulsion, enthaltend - bezogen auf ihr Gewicht -
a) mindestens 40 Gew.-% Wasser,
b) mindestens 0,5 Gew.-% mindestens eines primären, linearen, gesättigten C₁₄₋₃₀-Fettalkohols,
c) mindestens eine mehrschichtige Pigmentkapsel, umfassend
i) einen Partikelkern,
ii) eine diesen Partikelkern umgebende erste Hülle, welche - bezogen auf ihr Gewicht - mindestens 50 Gew.-% farbige Pigmente enthält,
iii) eine die erste Hülle umgebende zweite Hülle, welche - bezogen auf ihr Gewicht - mindestens 50 Gew.-% Titandioxid enthält,
iv) eine die zweite Hülle umgebende dritte Hülle aus einem wasserunlöslichen, thermoplastischem Polymer,
**dadurch gekennzeichnet, dass**
- die Emulsion lamellare flüssig-kristalline Strukturen aufweist und
- der Partikelkern der mehrschichtigen Pigmentkapsel
- 20 bis 40 Gew.-% des Gesamt-Kapselgewichts ausmacht und
- zu mindestens 50 Gew.-% seines Gewichts aus Alditolen, vorzugsweise aus Mannitol, besteht.

2. Lamellare Öl-in-Wasser-Emulsion nach Anspruch 1, **dadurch gekennzeichnet, dass** sie mindestens einen Alkohol, ausgewählt aus Behenylalkohol und Arachidylalkohol sowie Mischungen hiervon, in einer Gesamtmenge von 0,5 bis 10 Gew.-%, vorzugsweise 0,6 bis 9 Gew.-%, weiter bevorzugt 0,7 bis 8 Gew.-%, noch weiter bevorzugt 0,8 bis 7 Gew.-%, besonders bevorzugt 0,9 bis 6 Gew.-% und insbesondere 1 bis 5 Gew.-%, jeweils bezogen auf das Gewicht der Emulsion, enthält.

3. Lamellare Öl-in-Wasser-Emulsion nach einem der Ansprüche 1 oder 2, **dadurch gekennzeichnet, dass** sie mindestens ein Salz von C₁₂₋₂₀-Alkylphosphaten, insbesondere mindestens ein Salz von Cetylphosphat, in einer Gesamtmenge von 0,5 bis 10 Gew.-%, vorzugsweise 0,6 bis 9 Gew.-%, weiter bevorzugt 0,7 bis 8 Gew.-%, noch weiter bevorzugt 0,8 bis 7 Gew.-%, besonders bevorzugt 0,9 bis 6 Gew.-% und insbesondere 1 bis 5 Gew.-%, jeweils bezogen auf das Gewicht der Emulsion, enthält.

4. Lamellare Öl-in-Wasser-Emulsion nach einem der Ansprüche 1 bis 3, **dadurch gekennzeichnet, dass** sie keine Mikroemulsion und keine PIT-Emulsion ist.

5. Lamellare Öl-in-Wasser-Emulsion nach einem der Ansprüche 1 bis 4, **dadurch gekennzeichnet, dass** der Partikelkern der mehrschichtigen Pigmentkapsel zu mindestens 60 Gew.-%, weiter bevorzugt zu mindestens 70 Gew.-% und insbesondere zu mindestens 75 Gew.-% seines Gewichts aus Alditolen, vorzugsweise aus Mannitol, besteht.

6. Lamellare Öl-in-Wasser-Emulsion nach einem der Ansprüche 1 bis 5, **dadurch gekennzeichnet, dass** sie mehrschichtige Pigmentkapseln enthält, welche - bezogen auf das Gewicht der Pigmentkapsel - 10 bis 40 Gew.-%, vorzugsweise 12,5 bis 37,5 Gew.-%, weiter bevorzugt 15 bis 35 Gew.-%, noch weiter bevorzugt 17,5 bis 32,5 Gew.-%, besonders bevorzugt 20 bis 30 Gew.-% und insbsondere 22,5 bis 27,5 Gew.-% Mannitol enthalten.

7. Lamellare Öl-in-Wasser-Emulsion nach einem der Ansprüche 1 bis 6, **dadurch gekennzeichnet, dass** die den Partikelkern umgebende erste Hülle
- 20 bis 40 Gew.-% des Gesamt-Kapselgewichts ausmacht und
- zu mindestens 50 Gew.-%, vorzugsweise zu mindestens 60 Gew.-%, weiter bevorzugt zu mindestens 70 Gew.-% und insbesondere zu mindestens 75 Gew.-% ihres Gewichts aus einem oder mehreren der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz) besteht.

8. Lamellare Öl-in-Wasser-Emulsion nach einem der Ansprüche 1 bis 7, **dadurch gekennzeichnet, dass** sie mehrschichtige Pigmentkapseln enthält, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbsondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz) enthalten.

9. Lamellare Öl-in-Wasser-Emulsion nach einem der Ansprüche 1 bis 8, **dadurch gekennzeichnet, dass** die die erste Hülle umgebende zweite Hülle
- 20 bis 40 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Titandioxid (Cl 77891) besteht.

10. Lamellare Öl-in-Wasser-Emulsion nach einem der Ansprüche 1 bis 9, **dadurch gekennzeichnet, dass** sie mehrschichtige Pigmentkapseln enthält, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz)
enthalten.

11. Lamellare Öl-in-Wasser-Emulsion nach einem der Ansprüche 1 bis 10, **dadurch gekennzeichnet, dass** die die zweite Hülle umgebende dritte Hülle
- 2 bis 7 Gew.-% des Gesamt-Kapselgewichts ausmacht und
- zu mindestens 50 Gew.-%, vorzugsweise zu mindestens 70 Gew.-%, weiter bevorzugt zu mindestens 85 Gew.-% und insbesondere zu mindestens 95 Gew.-% ihres Gewichts aus Polymethylmethacrylat besteht.

12. Lamellare Öl-in-Wasser-Emulsion nach einem der Ansprüche 1 bis 11, **dadurch gekennzeichnet, dass** sie mehrschichtige Pigmentkapseln enthält, welche - bezogen auf das Gewicht der Pigmentkapsel - 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% Titandioxid (Cl 77891) enthalten.

13. Lamellare Öl-in-Wasser-Emulsion nach einem der Ansprüche 1 bis 12, **dadurch gekennzeichnet, dass** sie mehrschichtige Pigmentkapseln enthält, welche - bezogen auf das Gewicht der Pigmentkapsel -
- 22,5 bis 27,5 Gew.-% Mannitol und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% eines oder mehrerer der Pigmente Pigment Green (Cl 10006 grün), Pigment Brown 1 (Cl 12480 braun), Chlorierte Phthalocyanine (Cl 74260 grün), Pigment Red 101 und 102 (Cl 77015 rot), Kohlenstoff (Cl 77266 schwarz), Pigment Black 9 (Cl 77267 schwarz), Pigment Blue 28, Pigment Green 14 (Cl 77346 grün), Pigment Metal 2 (Cl 77400 braun), Gold (Cl 77480 braun), Eisenoxide und -hydoxide (Cl 77489 orange), Eisenoxid (Cl 77491 rot), Eisenoxid (Cl 77499 schwarz) und
- 10 bis 40 Gew.-%, vorzugsweise 12,5 bis 38 Gew.-%, weiter bevorzugt 15 bis 36 Gew.-%, noch weiter bevorzugt 17,5 bis 34 Gew.-%, besonders bevorzugt 20 bis 32 Gew.-% und insbesondere 22,5 bis 30 Gew.-% Titandioxid (Cl 77891)
enthalten.

14. Lamellare Öl-in-Wasser-Emulsion nach einem der Ansprüche 1 bis 13, **dadurch gekennzeichnet, dass** mindestens 90 Gew.-% der mehrschichtigen Pigmentkapseln eine Partikelgröße von 75 nm bis 211 µm (70 bis 200 Mesh) aufweisen.

15. Verwendung von mehrschichtigen Pigmentkapseln, umfassend
i) einen Partikelkern,
ii) eine diesen Partikelkern umgebende erste Hülle, welche - bezogen auf ihr Gewicht - mindestens 50 Gew.-% farbige Pigmente enthält,
iii) eine die erste Hülle umgebende zweite Hülle, welche - bezogen auf ihr Gewicht - mindestens 50 Gew.-% Titandioxid enthält,
iv) eine die zweite Hülle umgebende dritte Hülle aus einem wasserunlöslichen, thermoplastischem Polymer,
in O/W-Emulsionen mit lamellaren flüssig-kristallinen Strukturen.

## Claims

1. An oil-in-water emulsion, containing, based on its weight,
a) at least 40 wt.% water,
b) at least 0.5 wt.% of at least one primary, linear, saturated C₁₄₋₃₀ fatty alcohol,
c) at least one multilayer pigment capsule, comprising
i) a particle core,
ii) a first shell that surrounds said particle core and contains, based on its weight, at least 50 wt.% colored pigments,
iii) a second shell that surrounds the first shell and contains, based on its weight, at least 50 wt.% titanium dioxide,
iv) a third shell that surrounds the second shell and is made of a water-insoluble, thermoplastic polymer,
**characterized in that**
- the emulsion comprises lamellar liquid-crystal structures, and
- the particle core of the multilayer pigment capsule
- makes up 20 to 40 wt.% of the total weight of the capsule, and
- consists, in a proportion of at least 50 wt.% of its weight, of alditols, preferably of mannitol.

2. The lamellar oil-in-water emulsion according to claim 1, **characterized in that** it contains at least one alcohol selected from behenyl alcohol and arachidyl alcohol and mixtures thereof, in a total amount of from 0.5 to 10 wt.%, preferably 0.6 to 9 wt.%, more preferably 0.7 to 8 wt.%, even more preferably 0.8 to 7 wt.%, particularly preferably 0.9 to 6 wt.%, and in particular 1 to 5 wt.%, in each case based on the weight of the emulsion.

3. The lamellar oil-in-water emulsion according to one of claim 1 or 2, **characterized in that** it contains at least one salt of C₁₂₋₂₀ alkyl phosphates, in particular at least one salt of cetyl phosphate, in a total amount of from 0.5 to 10 wt.%, preferably 0.6 to 9 wt.%, more preferably 0.7 to 8 wt.%, even more preferably 0.8 to 7 wt.%, particularly preferably 0.9 to 6 wt.%, and in particular 1 to 5 wt.%, in each case based on the weight of the emulsion.

4. The lamellar oil-in-water emulsion according to one of claims 1 to 3, **characterized in that** it is not a microemulsion or a PIT emulsion.

5. The lamellar oil-in-water emulsion according to one of claims 1 to 4, **characterized in that** the particle core of the multilayer pigment capsule consists, in a proportion of at least 60 wt.%, more preferably in a proportion of at least 70 wt.%, and in particular in a proportion of at least 75 wt.% of its weight, of alditols, preferably of mannitol.

6. The lamellar oil-in-water emulsion according to one of claims 1 to 5, **characterized in that** it contains multilayer pigment capsules which contain, based on the weight of the pigment capsule, 10 to 40 wt.%, preferably 12.5 to 37.5 wt.%, more preferably 15 to 35 wt.%, even more preferably 17.5 to 32.5 wt.%, particularly preferably 20 to 30 wt.%, and in particular 22.5 to 27.5 wt.% mannitol.

7. The lamellar oil-in-water emulsion according to one of claims 1 to 6, **characterized in that** the first shell that surrounds the particle core
- makes up 20 to 40 wt.% of the total weight of the capsule, and
- consists, in a proportion of at least 50 wt.%, preferably in a proportion of at least 60 wt.%, more preferably in a proportion of at least 70 wt.%, and in particular in a proportion of at least 75 wt.% of its weight, of one or more of the pigments Pigment Green (Cl 10006 green), Pigment Brown 1 (Cl 12480 brown), chlorinated phthalocyanine (Cl 74260 green), Pigment Red 101 and 102 (Cl 77015 red), Carbon (Cl 77266 black), Pigment Black 9 (Cl 77267 black), Pigment Blue 28, Pigment Green 14 (Cl 77346 green), Pigment Metal 2 (Cl 77400 brown), Gold (Cl 77480 brown), iron oxides and hydroxides (Cl 77489 orange), iron oxide (Cl 77491 red), iron oxide (Cl 77499 black).

8. The lamellar oil-in-water emulsion according to one of claims 1 to 7, **characterized in that** it contains multilayer pigment capsules which contain, based on the weight of the pigment capsule, 10 to 40 wt.%, preferably 12.5 to 38 wt.%, more preferably 15 to 36 wt.%, even more preferably 17.5 to 34 wt.%, particularly preferably 20 to 32 wt.%, and in particular 22.5 to 30 wt.% of one or more of the pigments Pigment Green (Cl 10006 green), Pigment Brown 1 (Cl 12480 brown), chlorinated phthalocyanine (Cl 74260 green), Pigment Red 101 and 102 (Cl 77015 red), Carbon (Cl 77266 black), Pigment Black 9 (Cl 77267 black), Pigment Blue 28, Pigment Green 14 (Cl 77346 green), Pigment Metal 2 (Cl 77400 brown), Gold (Cl 77480 brown), iron oxides and hydroxides (Cl 77489 orange), iron oxide (Cl 77491 red), iron oxide (Cl 77499 black).

9. The lamellar oil-in-water emulsion according to one of claims 1 to 8, **characterized in that** the second shell that surrounds the first shell
- makes up 20 to 40 wt.% of the total weight of the capsule, and
- consists, in a proportion of at least 50 wt.%, preferably in a proportion of at least 70 wt.%, more preferably in a proportion of at least 85 wt.%, and in particular in a proportion of at least 95 wt.% of its weight, of titanium dioxide (Cl 77891).

10. The lamellar oil-in-water emulsion according to one of claims 1 to 9, **characterized in that** it contains multilayer pigment capsules which contain, based on the weight of the pigment capsule,
- 22.5 to 27.5 wt.% mannitol, and
- 10 to 40 wt.%, preferably 12.5 to 38 wt.%, more preferably 15 to 36 wt.%, even more preferably 17.5 to 34 wt.%, particularly preferably 20 to 32 wt.%, and in particular 22.5 to 30 wt.% of one or more of the pigments Pigment Green (Cl 10006 green), Pigment Brown 1 ((Cl 12480 brown), chlorinated phthalocyanine (Cl 74260 green), Pigment Red 101 and 102 (Cl 77015 red), Carbon (Cl 77266 black), Pigment Black 9 (Cl 77267 black), Pigment Blue 28, Pigment Green 14 (Cl 77346 green), Pigment Metal 2 (Cl 77400 brown), Gold (Cl 77480 brown), iron oxides and hydroxides (Cl 77489 orange), iron oxide (Cl 77491 red), iron oxide (Cl 77499 black).

11. The lamellar oil-in-water emulsion according to one of claims 1 to 10, **characterized in that** the third shell that surrounds the second shell
- makes up 2 to 7 wt.% of the total weight of the capsule, and
- consists, in a proportion of at least 50 wt.%, preferably in a proportion of at least 70 wt.%, more preferably in a proportion of at least 85 wt.%, and in particular in a proportion of at least 95 wt.% of its weight, of polymethyl methacrylate.

12. The lamellar oil-in-water emulsion according to one of claims 1 to 11, **characterized in that** it contains multilayer pigment capsules which contain, based on the weight of the pigment capsule, 10 to 40 wt.%, preferably 12.5 to 38 wt.%, more preferably 15 to 36 wt.%, even more preferably 17.5 to 34 wt.%, particularly preferably 20 to 32 wt.%, and in particular 22.5 to 30 wt.% titanium dioxide (Cl 77891).

13. The lamellar oil-in-water emulsion according to one of claims 1 to 12, **characterized in that** it contains multilayer pigment capsules which contain, based on the weight of the pigment capsule,
- 22.5 to 27.5 wt.% mannitol, and
- 10 to 40 wt.%, preferably 12.5 to 38 wt.%, more preferably 15 to 36 wt.%, even more preferably 17.5 to 34 wt.%, particularly preferably 20 to 32 wt.%, and in particular 22.5 to 30 wt.% of one or more of the pigments Pigment Green (Cl 10006 green), Pigment Brown 1 ((Cl 12480 brown), chlorinated phthalocyanine (Cl 74260 green), Pigment Red 101 and 102 (Cl 77015 red), Carbon (Cl 77266 black), Pigment Black 9 (Cl 77267 black), Pigment Blue 28, Pigment Green 14 (Cl 77346 green), Pigment Metal 2 (Cl 77400 brown), Gold (Cl 77480 brown), iron oxides and hydroxides (Cl 77489 orange), iron oxide (Cl 77491 red), iron oxide (Cl 77499 black), and
- 10 to 40 wt.%, preferably 12.5 to 38 wt.%, more preferably 15 to 36 wt.%, even more preferably 17.5 to 34 wt.%, particularly preferably 20 to 32 wt.%, and in particular 22.5 to 30 wt.% titanium dioxide (Cl 77891).

14. The lamellar oil-in-water emulsion according to one of claims 1 to 13, **characterized in that** at least 90 wt.% of the multilayer pigment capsules have a particle size of from 75 nm to 211 µm (70 to 200 mesh).

15. The use of multilayer pigment capsules, comprising
i) a particle core,
ii) a first shell that surrounds said particle core and contains, based on its weight, at least 50 wt.% colored pigments,
iii) a second shell that surrounds the first shell and contains, based on its weight, at least 50 wt.% titanium dioxide,
iv) a third shell that surrounds the second shell and is made of a water-insoluble, thermoplastic polymer,
in O/W emulsions having lamellar liquid-crystal structures.

## Revendications

1. Émulsion huile-dans-l'eau, contenant - rapporté à son poids -
a) au moins 40 % en poids en eau,
b) au moins 0,5 % en poids d'au moins un alcool gras C₁₄₋₃₀ primaire, linéaire, saturé,
c) au moins une capsule de pigment multicouche comprenant
i) un noyau de particule,
ii) une première enveloppe entourant ce noyau de particule, laquelle contient - rapporté à son poids - au moins 50 % en poids de pigments colorés,
iii) une deuxième enveloppe entourant la première enveloppe, laquelle contient - rapporté à son poids - au moins 50 % en poids de dioxyde de titane,
iv) une troisième enveloppe entourant la deuxième enveloppe, constituée d'un polymère thermoplastique non-soluble dans l'eau,
**caractérisée en ce que**
- l'émulsion présente des structures cristallines liquides lamellaires et
- le noyau de particule de la capsule de pigment multicouche
- représente 20 à 40 % en poids du poids total de la capsule et
- dont le poids est constitué à au moins 50 % en poids d'alditols, préférablement de mannitol.

2. Émulsion huile-dans-l'eau lamellaire selon la revendication 1, **caractérisée en ce qu'**elle contient au moins un alcool, sélectionné parmi le groupe de l'alcool béhénylique et l'alcool arachidylique, ainsi que des composés de ceux-ci, dans une quantité totale de 0,5 à 10 % en poids, préférablement de 0,6 à 9 % en poids, plus préférablement de 0,7 à 8 % en poids, encore plus préférablement de 0,8 à 7 %, très préférablement de 0,9 à 6 % en poids et en particulier de 1 à 5 % en poids, respectivement rapporté au poids de l'émulsion.

3. Émulsion huile-dans-l'eau lamellaire selon l'une quelconque des revendications 1 ou 2, **caractérisée en ce qu'**elle contient au moins un sel de phosphate d'alkyle C₁₂₋₂₀, notamment au moins un sel de cétylphosphate, dans une quantité totale de 0,5 à 10 % en poids, préférablement de 0,6 à 9 % en poids, plus préférablement de 0,7 à 8 % en poids, encore plus préférablement de 0,8 à 7 % en poids, très préférablement de 0,9 à 6 % en poids et on ne peut plus préférablement de 1 à 5 % en poids, respectivement rapporté au poids de l'émulsion.

4. Émulsion huile-dans-l'eau lamellaire selon l'une quelconque des revendications 1 à 3, **caractérisée en ce qu'**elle n'est pas une microémulsion et n'est pas une émulsion PIT (phase inversion temperature / température d'inversion de phase).

5. Émulsion huile-dans-l'eau lamellaire selon l'une quelconque des revendications 1 à 4, **caractérisée en ce que** le poids du noyau de particule de la capsule de pigments multicouche est constitué d'au moins 60 % en poids, plus préférablement d'au moins 70 % en poids et encore plus préférablement d'au moins 75 % en poids d'alditols, préférablement de mannitol.

6. Émulsion huile-dans-l'eau lamellaire selon l'une quelconque des revendications 1 à 5, **caractérisée en ce qu'**elle contient des capsules de pigment multicouches qui contiennent-rapporté au poids de la capsule de pigment - 10 à 40 % en poids, préférablement de 12,5 à 37,5 % en poids, plus préférablement de 15 à 35 % en poids, encore plus préférablement de 17,5 à 32,5 % en poids, très préférablement de 20 à 30 % en poids et on ne peut plus préférablement de 22,5 à 27,5 % en poids de mannitol.

7. Émulsion huile-dans-l'eau lamellaire selon l'une quelconque des revendications 1 à 6, **caractérisée en ce que** la première enveloppe entourant le noyau de particule
- représente 20 à 40 % en poids du poids total de la capsule et
- dont le poids est constitué à au moins 50 % en poids, préférablement à au moins 60 % en poids, plus préférablement 70 % en poids et on ne peut plus préférablement à au moins 75 % en poids d'un ou plusieurs des pigments Pigment Green (CI 10006 vert), Pigment Brown 1 (Cl 12480 marron), de phtalocyanine chlorée (Cl 74260 vert), Pigment Red 101 et 102 (Cl 77015 rouge), de carbone (Cl 77266 noir), Pigment Black 9 (Cl 77267 noir), Pigment Blue 28, Pigment Green 14 (Cl 77346 vert), Pigment Metal 2 (Cl 77400 marron), Gold (Cl 77480 marron), d'oxydes et d'hydroxydes de fer (Cl 77489 orange), d'oxyde de fer (CI 77491 rouge), d'oxyde de fer (CI 77499 noir).

8. Émulsion huile-dans-l'eau lamellaire selon l'une quelconque des revendications 1 à 7, **caractérisée en ce qu'**elle contient des capsules de pigment multicouche, qui contiennent - rapporté au poids de la capsule de pigment- 10 à 40 % en poids, préférablement de 12,5 à 38 % en poids, plus préférablement de 15 à 36 % en poids, encore plus préférablement de 17,5 à 34 % en poids, très préférablement de 20 à 32 % en poids et on ne peut plus préférablement de 22,5 à 30 % en poids d'un ou plusieurs des pigments Pigment Green (Cl 10006 vert), Pigment Brown 1 (Cl 12480 marron), de phtalocyanine chlorée (CI 74260 vert), Pigment Red 101 et 102 (CI 77015 rouge), de carbone (CI 77266 noir), Pigment Black 9 (Cl 77267 noir), Pigment Blue 28, Pigment Green 14 (Cl 77346 vert), Pigment Metal 2 (Cl 77400 marron), Gold (CI 77480 marron), d'oxydes et d'hydroxydes de fer (CI 77489 orange), d'oxyde de fer (CI 77491 rouge), d'oxyde de fer (CI 77499 noir).

9. Émulsion huile-dans-l'eau lamellaire selon l'une quelconque des revendications 1 à 8, **caractérisée en ce que** la deuxième enveloppe entourant la première enveloppe
- représente 20 à 40 % en poids du poids total de la capsule et
- dont le poids est constitué à au moins 50 % en poids, préférablement à au moins 70 % en poids, plus préférablement à au moins 85 % en poids et on ne peut plus préférablement à au moins 95 % en poids de dioxyde de titane (CI 77891).

10. Émulsion huile-dans-l'eau lamellaire selon l'une quelconque des revendications 1 à 9, **caractérisée en ce qu'**elle contient des capsules de pigment multicouche, qui contiennent - rapporté au poids de la capsule de pigment-
- 22,5 à 27,5 % en poids de mannitol et
- 10 à 40 % en poids, préférablement de 12,5 à 38 % en poids, plus préférablement de 15 à 36 % en poids, encore plus préférablement de 17,5 à 34% en poids, très préférablement de 20 à 32 % en poids et on ne peut plus préférablement de 22,5 à 30 % en poids d'un ou plusieurs des pigments Pigment Green (Cl 10006 vert), Pigment Brown 1 (CI 12480 marron), de phtalocyanine chlorée (CI 74260 vert), Pigment Red 101 et 102 (CI 77015 rouge), de carbone (CI 77266 noir), Pigment Black 9 (CI 77267 noir), Pigment Blue 28, Pigment Green 14 (CI 77346 vert), Pigment Metal 2 (Cl 77400 marron), Gold (Cl 77480 marron), d'oxydes et d'hydroxydes de fer (Cl 77489 orange), d'oxyde de fer (Cl 77491 rouge), d'oxyde de fer (Cl 77499 noir).

11. Émulsion huile-dans-l'eau lamellaire selon l'une quelconque des revendications 1 à 10, **caractérisée en ce que** la troisième enveloppe entourant la deuxième enveloppe
- représente 2 à 7 % en poids du poids total de la capsule et
- dont le poids est constitué à au moins 50 % en poids, préférablement à au moins 70 % en poids, plus préférablement à au moins 85 % en poids et on ne peut plus préférablement à au moins 95 % en poids en polyméthacrylate de méthyle.

12. Émulsion huile-dans-l'eau lamellaire selon l'une quelconque des revendications 1 à 11, **caractérisée en ce qu'**elle contient des capsules de pigment multicouche, qui contiennent - rapporté au poids de la capsule de pigment- de 10 à 40 % en poids, préférablement de 12,5 à 38 % en poids, plus préférablement de 15 à 36 % en poids, encore plus préférablement de 17,5 à 34 % en poids, très préférablement de 20 à 32 % en poids et on ne peut plus préférablement de 22,5 à 30 % en poids de dioxyde de titane (CI 77891).

13. Émulsion huile-dans-l'eau lamellaire selon l'une quelconque des revendications 1 à 12, **caractérisée en ce qu'**elle contient des capsules de pigment multicouche, qui contiennent - rapporté au poids de la capsule de pigment-
- 22,5 à 27,5 % en poids de mannitol et
- de 10 à 40 % en poids, préférablement de 12,5 à 38 % en poids, plus préférablement de 15 à 36 % en poids, encore plus préférablement de 17,5 à 34% en poids, très préférablement de 20 à 32 % en poids, et on ne peut plus préférablement de 22,5 à 30 % en poids d'un ou plusieurs des pigments Pigment Green (Cl 10006 vert), Pigment Brown 1 (CI 12480 marron), de phtalocyanine chlorée (CI 74260 vert), Pigment Red 101 et 102 (Cl 77015 rouge), de carbone (Cl 77266 noir), Pigment Black 9 (Cl 77267 noir), Pigment Blue 28, Pigment Green 14 (Cl 77346 vert), Pigment Metal 2 (Cl 77400 marron), Gold (CI 77480 marron), d'oxydes et d'hydroxydes de fer (Cl 77489 orange), d'oxyde de fer (CI 77491 rouge), d'oxyde de fer (CI 77499 noir) et
- de 10 à 40 % en poids, préférablement de 12,5 à 38 % en poids, plus préférablement de 15 à 36 % en poids, encore plus préférablement de 17,5 à 34% en poids, très préférablement de 20 à 32 % en poids et on ne peut plus préférablement de 22,5 à 30 % en poids en dioxyde de titane (CI 77891).

14. Émulsion huile-dans-l'eau lamellaire selon l'une quelconque des revendications 1 à 13, **caractérisée en ce qu'**au moins 90 % en poids des capsules de pigment multicouches présentent une dimension des particules de 75 nm à 211 µm (maille de 70 à 200).

15. Utilisation de capsules de pigments multicouche comprenant
i) un noyau de particule,
ii) une première enveloppe entourant ce noyau de particule, qui contient - rapporté à son poids - au moins 50 % en poids de pigments colorés,
iii) une deuxième enveloppe entourant la première enveloppe, qui contient - rapporté à son poids - au moins 50 % en poids de dioxyde de titane,
iv) une troisième enveloppe entourant la deuxième enveloppe constituée d'un polymère thermoplastique non-soluble dans l'eau,
dans des émulsions huile-dans-l'eau comportant des structures cristallines fluides lamellaires.
